(19) Europäisches Patentamt
European Patent Office
Office européen des brevets

(11) Veröffentlichungsnummer: **0 170 863**
**A1**

# (12) EUROPÄISCHE PATENTANMELDUNG

(21) Anmeldenummer: **85107969.9**

(22) Anmeldetag: **27.06.85**

(51) Int. Cl.[4]: **C 07 C 101/02**, C 07 C 87/127, C 07 C 87/24, A 61 K 7/46

(30) Priorität: **11.07.84 CH 3361/84**
**10.05.85 CH 2002/85**

(43) Veröffentlichungstag der Anmeldung: **12.02.86**
**Patentblatt 86/7**

(84) Benannte Vertragsstaaten: **CH DE FR GB LI NL**

(71) Anmelder: **L. GIVAUDAN & CIE Société Anonyme, CH-1214 Vernier-Genève (CH)**

(72) Erfinder: **De Polo, Karl Fred, 35 avenue du Gros-Chêne, CH-1213 Onex (CH)**
Erfinder: **Ochsner, Paul Albert, 30 avenue des Tilleuls, CH-1213 Genf (CH)**
Erfinder: **Pittet, Gilbert H., 10 chemin de la Source, CH-1296 Coppet (CH)**

(74) Vertreter: **Urech, Peter, Dr. et al, Grenzacherstrasse 124 Postfach 3255, CH-4002 Basel (CH)**

## (54) Neue Salze sekundärer und tertiärer aliphatischer Amine mit Aminopolycarbonsäuren.

(57) Neue, in alkoholischer Lösung praktisch neutral reagierende Salze sekundärer di-$C_{6-22}$-Alkylamine und insbesondere tertiärer $C_{16-22}$-Alkyl-dimethyl- und di-$C_{6-22}$-Alkyl-$C_{1-}$-alkylamine mit Aminopolycarbonsäuren.

Riechstoffkompositionen mit einem Gehalt an solchen Salzen, Verfahren zur Herstellung dieser neuen Salze, Verfahren zur Stabilisierung von Riechstoffkompositionen unter Verwendung der neuen Salze, Verwendung der neuen Salze zur Stabilisierung von Riechstoffkompositionen.

EP 0 170 863 A1

1

Ref. 6510/217

L. Givaudan & Cie Société Anonyme, Vernier-Genève (Schweiz)

# Neue Salze sekundärer und tertiärer aliphatischer Amine mit Aminopolycarbonsäuren

Die Erfindung betrifft neue, in alkoholischer Lösung (praktisch) neutral reagierende Salze sekundärer di-$C_{6-22}$-Alkylamine, und insbesondere tertiärer $C_{16-22}$-Alkyl-dimethyl- und di-$C_{6-22}$-Alkyl-$C_{1-2}$-alkylamine mit Aminopolycarbonsäuren.

Die Erfindung betrifft auch Riechstoffkompositionen mit einem Gehalt an solchen Salzen. Die Erfindung betrifft ferner ein Verfahren zur Herstellung der neuen Salze, ein Verfahren zur Stabilisierung von Riechstoffkompositionen unter Verwendung der neuen erfindungsgemässen Salze und schliesslich die Verwendung der neuen erfindungsgemässen

Salze zur Stabilisierung von Riechstoffkompositionen.

Beispiele von definitionsgemässen sekundären, und insbesondere tertiären Aminen sind insbesondere tertiäre Fettamine, siehe z.B.

- Ullmann's Encyklopädie der technischen Chemie, 4. neubearbeitete und erweiterte Auflage, Chemie Verlag, Weinheim, Band 11, (1976), Seiten 447 - 452,

- Kirk Othmer, Encyclopedia of Chemical Technology, 3. Auflage, John Wiley & Sons, Inc. New York, Band 2, (1978), Seiten 283 - 295,

- The Chemistry of Fatty Amines, Armour Chemical Division, Armour and Company, Chicago, 1948.

Solche tertiären Fettamine sind zwar vorzugsweise geradkettig; sie können aber auch verzweigt oder cycloaliphatisch sein; sie können gesättigt oder ungesättigt sein.

Beispiele sind $C_{16-22}$-Alkyldimethylamine der Formeln

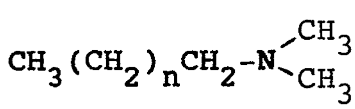

n insbesondere 16

Alkyl-dimethylamin "ADMA"

Octadecyldimethylamin "ADMA 8" Ethyl Corporation

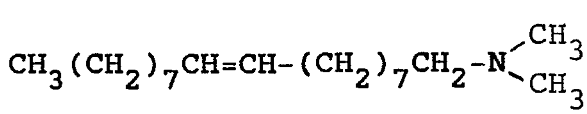

Oleyl-dimethylamin

"Crodamine 3 AOD" Croda Universal Ltd.

0170863

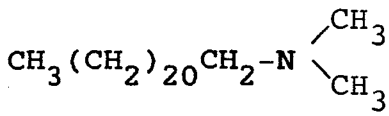

Behenyldimethylamin "Crodamine 3 ABD"

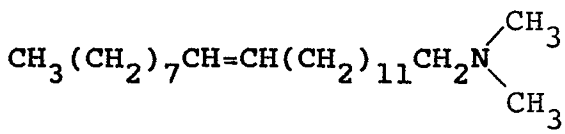

Erucyldimethylamin "Crodamine 3 AED"

Ein weiteres tertiäres Amin ist das

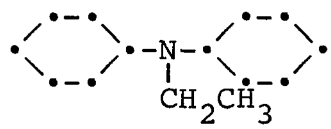

N-Aethyl-dicyclohexylamin

Beispiele von sekundären Aminen sind:

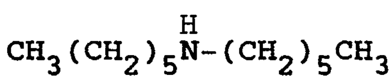

Dihexylamin

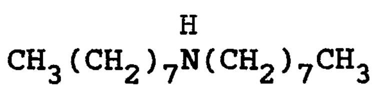

Dioctylamin

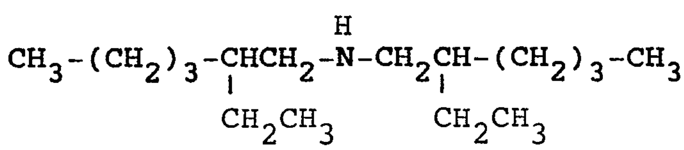

Diisooctylamin (Di-(2-äthylhexylamin))

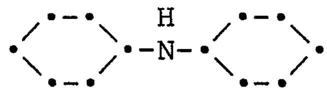

Dicyclohexylamin

$$CH_3(CH_2)_{17}-\overset{H}{N}-(CH_2)_{17}CH_3$$

Distearylamin (DSA)

Als Aminopolycarbonsäuren werden insbesondere die Komplexone, d.h. die als Komplexbildner (Chelatbildner) bekannten Substanzen verwendet, die mit zwei- und mehrwertigen Metallionen unter Komplexbildung Ringverbindungen bilden. Die diesbezüglich bevorzugten Säuren sind:

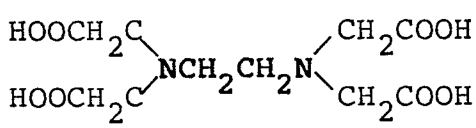

AeDTE

(Aethylendiamintetraessigsäure)

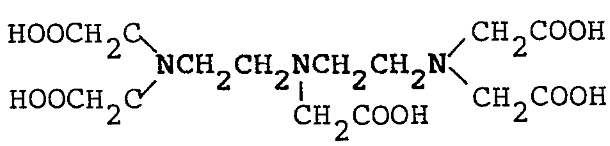

DTPE

(Diäthylentriaminpentaessigsäure)

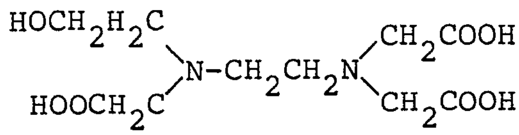

HAeDTE

(N-(2-Hydroxyäthyl)äthylendiamintriessigsäure)

Die Stabilisierung soll insbesondere die in Folge von Denaturierung und Zersetzung von Riechstoffkompositionen, z.B. bei der Lagerung entstehende, exzessiver Verfärbung verhindern. Die Verfärbung geht zudem oft mit einer Veränderung der Geruchseigenschaften einher, was sich natürlich im Falle von Riechstoffkompositionen sehr nachteilig auswirkt. Die Ursache von Denaturierung und Zersetzung kann vielfältig sein, z.B. Lichteinfluss, und/oder Oxidation, insbesondere katalysiert durch Anwesenheit von Schwermetallen, wie Eisen, Kupfer, etc., übermässige Acidität oder Basizität.

Im Falle der - im Vordergrund des Interesses stehenden - durch Schwermetallspuren verursachten Färbung von Riechstoffkompositionen ist diese Färbung sehr oft durch Komplexbildung des Schwermetallions mit Liganden des Riechstoffmoleküls bedingt. Solche Liganden sind insbesondere $O^{2-}$ und $OH^-$.

Erfindungsgemäss können nun diese Nachteile durch Verwendung der neuen Salze verhindert bzw. (grösstenteils) beseitigt werden; insbesondere können exzessive Färbungen so verhindert, bzw. solche Lösungen entfärbt, bzw. aufgehellt werden. Diese Entfärbung bzw. Aufhellung ist darum von Bedeutung, weil der Kunde in der Regel stark gefärbte Riechstoffkompositionen nicht ohne weiteres akzeptiert.

Die Herstellung der neuen Salze erfolgt in an sich bekannter Weise durch Neutralisation der nötigen Anzahl der sauren H-Atome des Komplexbildners mit dem Amin, üblicherweise sind dies 2 oder 3 der sauren H-Atome. Im Falle des - bevorzugten - AeDTE/ADMA 8 können die gebildeten neutralen Salze durch die folgenden Formeln wiedergegeben werden:

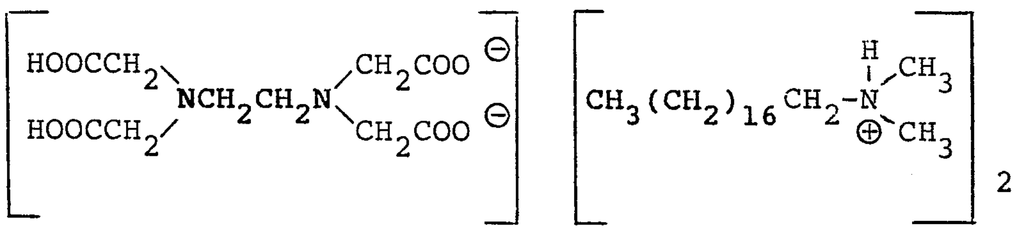
I'

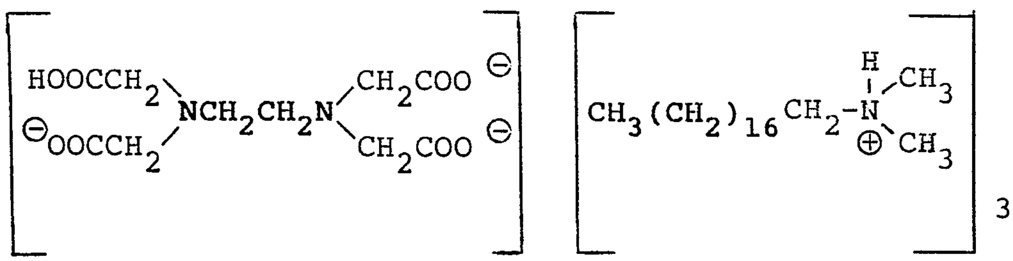
I''

Man arbeitet zweckmässigerweise in alkoholischer Lösung und zweckmässigerweise bei erhöhter Temperatur, z.B. bei 50°C bis 100°C. Durch Abkühlenlassen der neutralisierten Lösung kristallisieren die erfindungsgemässen Salze üblicherweise in reiner Form aus und können auf diese Weise leicht abgetrennt werden. Die neuen Salze können aber auch durch simples Abdampfen des Lösungsmittels als Rückstand in recht reiner Form gewonnen werden.

Eine 30%ige Lösung von I' in Aethanol weist einen pH-Wert von 5,5 auf.

Eine 30%ige Lösung (die Lösung enthält etwas Rückstand) von I'' in Aethanol weist einen pH-Wert von 6,5-7,0 auf.

Unter den Begriff "(praktisch) neutral reagierende Salze" sollen also auch noch schwach sauer reagierende Produkte fallen.

Zur erfindungsgemässen Stabilisierung sind pH-Werte von ca. 5,5 bis 7,5, insbesondere von 6,5 bis 7,0 angezeigt.

Ein weiteres bevorzugtes erfindungsgemässes Salz ist das:

AeDTE $(DSA)_3$

Weitere repräsentative Vertreter sind:

DTPE $(ADMA\ 8)_2$
DTPE $(ADMA\ 8)_3$
HAeDTPE $(ADMA\ 8)_2$.

Der Begriff "Riechstoffkomposition" soll im Rahmen der vorliegenden Erfindung breit verstanden werden. Er umfasst also Rohmaterialien und Endprodukte, und zwar natürliche und/oder synthetische Produkte; also z.B. ätherische Oele und/oder synthetisch chemische Stoffe und deren Gemische mit Lösungsmitteln, Träger- und Hilfsstoffen; er umfasst aber auch "Endprodukte", d.h. parfümierte - und stabilisierte - Gegenstände.

Beispiele von ätherischen Oelen sind insbesondere Bayöl, Bergamotteöl, Citronellaöl, Lemongrasöl, Mandarinenöl, Nelkenöl, Orangenöl, Patchouliöl, Vetiveröl, Eichenmoosextrakte, Castoreum, Ciste labdanum, Jasmin absolue, Cassis absolue, Narzissen absolue, Verveine absolue, usw.

Beispiele von Synthetika sind: Benzylsalicylat, Vanillin, Eugenol, Creosol, Chavikol, etc.

Der Begriff Riechstoffkompositionen umfasst insbesondere auch solche Riechstoffkompositionen, die Hilfsstoffe enthalten, die aufgrund ihrer Struktur - z.B. Anwesenheit von $O^{2-}$ oder $OH^-$ Liganden - starkes Komplexbildungsvermögen mit Schwermetallionen aufweisen, d.h. in ursprünglich nur schwachen oder gar nicht gefärbten Kompositionen unliebsame, exzessive Färbungen verursachen. Ein Beispiel eines solchen Hilfsstoffes ist das 4-(1,1-Dimethyläthyl)-4'-methoxydibenzoylmethan. Diese Substanz wird z.B. Riechstoffkompositionen zwecks Verhinderung phototoxischer Reaktionen zugesetzt, siehe z.B. die Australische Patentanmeldung Nr. 24450 vom 10. Februar 1984, veröffentlicht am 23. August 1984.

Die mit den erfindungsgemässen Salzen stabilisierten Riechstoffkompositionen enthalten oft vorzugsweise noch weitere Hilfsstoffe, nämlich konventionelle Antioxydantien, wie z.B. Fettsäureester der Ascorbinsäure, z.B. Ascorbylpalmitat, Butylhydroxyanisol, Butylhydroxytoluol, p-Methoxyphenol, Cetylgallat, Octylgallat, Dodecylgallat, Propylgallat, Tocopherole oder deren Ester, z.B. die Acetate, o-Toluylbiguanid, etc.
Die Verwendung solcher Antioxidantien oder Kombinationen davon ist bekannt, siehe z.B.

Roche Antioxidants (Ronoxan) von K. Nagy, Roche Information Service, Food Industries Department, 1984,

Roche Antioxidants, Technical Information on Ascorbyl Palmitate, dl-α-Tocopherol and Antioxidant Mixtures, P. Schuler, Roche Information Service, Food Industries Department, 1983,

Roche Products for Use in Pharmaceuticals and Cosmetics, 1985/86, Food and Pharmaceutical Industries Department, Vitamin and Fine Chemical Division, 1985,

Roche Products for the Food Industry, 1985/86, Food and Pharmaceutical Industries Department, Vitamin and Fine Chemical Division, 1985,

Vitamin E: Essential Nutrient, Exceptional Properties, Exciting Opportunities, Food and Pharmaceutical Industries Department, Vitamin and Fine Chemical Division, 1984 and

Autoxidation of Fats and its Prevention with Antioxidants, P. Schuler, Roche Information Service, Food Industries Department, 1980.

Ein zweckmässiges Gemisch enthält z.B.

| | Gewichtsteile |
|---|---|
| α-Tocopherol 50% | 20 |
| Ascorbylpalmitat | 50 |
| 4-(1,1-Dimethyläthyl)-4'-methoxydibenzoylmethan | 15 |
| AeDTE $(ADMA\ 8)_2$ | 15 |

Gemische dieser - und analoger - Art schützen Riechstoffkompositionen vor schädlichem Lichteinfluss, Luftsauerstoff und Verfärbung.

Die behandelten Riechstoffkompositionen können in flüssiger, pastenförmiger oder fester Form vorliegen. Dasselbe gilt für die oben beschriebenen Stabilisierungsgemische.

Die erfindungsgemäss verwendeten Salze können in einem Mengenverhältnis eingesetzt werden, das über einen weiten Bereich hin variiert; es ist jeweils abhängig von der Art der Riechstoffkomposition, der diese Salze zugegeben werden und insbesondere von der vorhandenen Menge an Schwermetallen. Ueblicherweise können sie in Konzentrationen von etwa 0,1 bis 10 Gew.%, bezogen auf das Gesamtgewicht der Riechstoffbestandteile eingesetzt werden. Die zweckmässigen Konzentrationen im Falle von Parfümkonzentraten sind etwa 2 bis 10 Gew.% und im Falle von verdünnten alkoholischen Lösungen 0,1 bis 1%, insbesondere 0,5%.

Die Salze I sind in absolutem Alkohol bzw. auch anderen polaren organischen Lösungsmitteln gut löslich. Zudem sind sie ausreichend fettlöslich.

Die Zugabe des Produktes erfolgt zweckmässigerweise als kristallines Salz oder in Form einer alkoholischen Lösung dieses Salzes. Allfällige Niederschläge von unlöslichen komplexierten Schwermetallionen werden hierauf zweckmässigerweise durch Filtration entfernt.

Lipolösliche, d.h. fettlösliche Stabilisierungsmittel für Riechstoffkompositionen sind zwar bereits bekannt, siehe z.B. die DT-OS 2,822,525 vom 7. Dezember 1978. Es handelt sich auch hier um lipolösliche Komplexierungsmittel, z.B. Mischpolymere von Aethylen- und Propylenoxyd mit Aethylendiamin, um Derivate von Phosphor-, Phosphon- und Diphosphonsäure bzw. um organische Ester, die sich von Phosphor- oder Phosphonsäure und einem äthoxylierten Fettalkohol ableiten.

Im Vergleich zu den vorbekannten Stabilisierungsmitteln sind die neuen Mittel jedoch insbesondere lichtstabiler, d.h. während der Lagerung werden in den ursprünglich entfärbten bzw. aufgehellten Kompositionen nach

einiger Zeit unerwünschte, erneute Verfärbungen in viel geringerem Umfang auftreten. Diese Lichtstabilität wird üblicherweise durch mehrstündiges Bestrahlen der Parfümkomposition in Glasgefässen, z.B. mit einer 1100 Watt Xenonlampe ermittelt.

Für Stabilisierungszwecke wurde bislang auch Weinsäure eingesetzt. Diese Säure weist aber den Nachteil auf, dass sich bei der Lagerung der so behandelten Parfümkompositionen nach einiger Zeit unerwünschte Niederschläge oder Ausflockungen bilden können.

## Beispiel 1

In einen mit Rührer, Thermometer und Tropftrichter versehenen Rundkolben gibt man 14,6 g (0,05 Mol) Aethylendiamintetraessigsäure und 40 g Aethanol. Man erwärmt das Gemisch auf 80°C und lässt unter Rühren innerhalb 15 Minuten 29,5 g (0,1 Mol) Dimethyloctadecylamin (ADMA 8) zutropfen. Nach der Zugabe erwärmt man noch 2 Stunden unter Rühren am Rückfluss. Man destilliert das Aethanol im Wasserstrahlvakuum ab. Der Rückstand wird im Vakuum bei 40°C bis zur Gewichtskonstanz getrocknet. Man erhält 44 g AeDTE $(ADMA\ 8)_2$ farblose Kristalle vom Schmelzintervall 68-76°C.

## Beispiel 2

In einen mit Rührer, Thermometer und Tropftrichter versehenen Rundkolben gibt man 29,2 g (0,1 Mol) Aethylendiamintetraessigsäure und 80 g Aethanol. Man erwärmt das Gemisch auf 80°C und lässt unter Rühren innerhalb 15 Minuten 89,0 g (0,3 Mol) Dimethyloctadecylamin (ADMA 8) zutropfen. Nach der Zugabe erwärmt man noch 2 Stunden unter Rühren am Rückfluss. Man destilliert das Aethanol im Wasserstrahlvakuum ab. Der Rückstand wird im Vakuum

getrocknet. Man erhält 85,2 g AeDTE (ADMA 8)$_3$, farblose Kristalle vom Schmelzintervall 68 - 74°C.

Beispiel 3

In einen mit Rührer, Thermometer und Tropftrichter versehenen Rundkolben gibt man 4,4 g (0,015 Mol) Aethylendiamintetraessigsäure und 30 g Aethanol. Man erwärmt das Gemisch auf 80°C und lässt unter Rühren innerhalb 15 Minuten 23,5 g (0,045 Mol) Distearylamin (DSA) zutropfen. Nach der Zugabe erwärmt man noch 2 Stunden unter Rühren am Rückfluss. Man destilliert das Aethanol im Wasserstrahlvakuum ab. Der Rückstand wird im Vakuum getrocknet. Man erhält 27,5 g AeDTE (DSA)$_3$ farblose Kristalle vom Schmelzintervall 83 - 100°C.

Beispiel 4

In einen mit Rührer, Thermometer und Tropftrichter versehenen Rundkolben gibt man 3,9 g (0,01 Mol) Diäthylentriaminpentaessigsäure (DTPE) und 30 g Aethanol. Man erwärmt das Gemisch auf 80°C und lässt unter Rühren innerhalb 15 Minuten 6,0 g (0,02 Mol) Dimethyloctadecylamin (ADMA 8) zutropfen. Nach der Zugabe erwärmt man noch 2 Stunden unter Rühren am Rückfluss. Man destilliert das Aethanol im Wasserstrahlvakuum ab. Der Rückstand wird im Vakuum getrocknet. Man erhält 9,9 g DTPE (ADMA 8)$_2$ farblose Kristalle vom Schmelzintervall 78 - 120°C.

Beispiel 5

In einen mit Rührer, Thermometer und Tropftrichter versehenen Rundkolben gibt man 4,2 g (0,015 Mol) N-(2-Hydroxyäthyl)äthylendiamintriessigsäure und 30 ml Aethanol. Man erwärmt das Gemisch auf 80°C und lässt unter Rühren innerhalb ca. 15 Minuten 9 g (0,03 Mol) Dimethyloctadecylamin (ADMA 8) zutropfen. Nach der Zugabe erwärmt man noch 2 Stunden unter Rühren am Rückfluss. Man destilliert das Aethanol im Wasserstrahlvakuum ab. Der Rückstand wird im Vakuum getrocknet. Man erhält 13 g HAeDTE $(ADMA\ 8)_2$ als semi-kristallines, gelblich gefärbtes Produkt.

## Beispiel 6

In einen mit Rührer, Thermometer und Tropftrichter versehenen Rundkolben gibt man 3,9 g (0,01 Mol) Diäthylentriaminpentaessigsäure und 30 g Aethanol. Man erwärmt das Gemisch auf 80°C und lässt unter Rühren innerhalb 15 Minuten 8,9 g (0,03 Mol) Dimethyloctadecylamin (ADMA 8) zutropfen. Nach der Zugabe erwärmt man noch 2 Stunden unter Rühren am Rückfluss. Man destilliert das Aethanol im Wasserstrahlvakuum ab. Der Rückstand wird im Vakuum getrocknet. Man erhält 15,0 g DTPE $(ADMA\ 8)_3$ als farblose Kristalle vom Schmelzintervall 70 - 100°C.

Der Rückstand lässt sich - wie im Falle der Produkte der Beispiele 1 - 5 - ohne weitere Reinigung für die Stabilisierungszwecke verwenden.

<u>IR-Spektrum:</u> Im Falle der Salze der Beispiele 1 - 6 konnten die Strukturen auch durch die entsprechenden Gruppierungen $H-\overset{|}{\underset{|}{N}}-^{\oplus}$, $-COO^{\ominus}$, -COOH, $-CH_2OH$, -OH bestätigt werden; die Aufnahmen erfolgten mittels eines Infrarot-Spektrophotometers Perkin-Elmer 681 - Gerätes; Proben: Aufschlämmungen der Salze I in Nujol.

## Beispiel 7

100 g Benzylsalicylat, die mit Spuren Eisen verunreinigt sind und daher eine rosarote Farbe aufweisen, werden bei Zimmertemperatur mit 0,25 g AeDTE $(ADMA\ 8)_2$ versetzt. Nach einigen Minuten Rühren wird das Gemisch vollständig farblos. Wenn man in diesem Fall als Komplexierungsmittel AeDTE $(ADMA\ 8)_3$ einsetzt, wird ein analoges Resultat erzielt.

## Beispiel 8

Ein Muster handelsüblichen Patchouliöls enthält 560 ppm Fe und 1,5 ppm Cu.

Das Oel weist eine rotbraune Farbe auf, die nach vierstündiger Bestrahlung noch intensiver wird. [Nach vier weiteren Stunden Bestrahlung war ein Muster des Oels nahezu schwarz.]

Zugabe von 2% AeDTE $(ADMA\ 8)_2$ bzw. AeDTE $(ADMA\ 8)_3$ zum rotbraunen Oel, gefolgt von vierstündigem Rühren und gegebenenfalls Filtrieren, führt zu einer hellgelben Lösung. Achtstündige Bestrahlung der so entfärbten Lösung hat keine geruchliche oder farbliche Aenderung zur Folge.

Bestrahlung (Beispiel 8 und folgende): Mit Bestrahlungsapparatur "Suntest", Original HANAU, bei 37°C in 30 ml Glasbehältern mit Bakelit Schraubverschluss; Lichtquelle: 1100 Watt Xenonlampe (150 000 Lux).

## Beispiel 9

Das Patchouliöl des Beispiels 3 wird durch folgende Parfümkomposition, die eine ausgeprägte Tabak-Patchouli-Note aufweist, ersetzt:

| | Gewichtsteile |
|---|---|
| Römisches Kümmelöl (Cuminöl) | 1 |
| Gewürznelkenöl | 5 |
| Zimtöl (chinesisch) | 15 |
| Zimtalkohol synthetisch | 20 |
| Vetiveröl Bourbon | 20 |
| Crysolid® (Givaudan) (6-tert. Butyl-1,1-dimethyl-4-acetyl-indan) | 30 |
| 12-Oxahexadecanolid | 30 |
| Isoraldeine 70® (Isomethyl-α-ionon) | 40 |
| Geraniumöl (Afrika) | 50 |
| Eichenmoos (entfärbt) | 50 |
| Sandelholzöl | 70 |
| Coumarin rein | 100 |
| Dipropylenglycol | 114 |
| Amylsalicylat | 125 |
| Lavendelöl (Frankreich) | 200 |
| Patchouliöl | 130 |

Die obige Komposition weist eine dunkle, braun-rote Farbe auf, die durch Belichten noch intensiver wird. Der Zusatz von 2 Gew.% AeDTE $(ADMA\ 8)_2$ oder AeDTE $(DSA)_3$ hellt die Komposition nach wenigen Minuten zu einer hellen, caramelfarbenen Flüssigkeit auf. Vierstündiges Bestrahlen eines behandelten Musters erbringt weder eine farbliche noch eine geruchliche Veränderung.

Beispiel 10

Die Komposition des Beispiels 9 wird mit Aethylalkohol zu einer 10%igen Lösung verdünnt. Diese gelbbraune, alkoholische Lösung wird während 8 Stunden belichtet, wobei der Farbton noch intensiver wird.

Wenn zu 100 ml dieser alkoholischen Lösung 0,25 g AeDTE $(ADMA\ 8)_2$ zugegeben werden, entfärbt sie sich augenblicklich praktisch vollständig, d.h. die Lösung ist nur noch leicht gelblich gefärbt. Vierstündige Bestrahlung dieser Lösung erbringt keine geruchliche oder farbliche Veränderung. Wenn man anstelle von AeDTE $(ADMA\ 8)_2$ 0,25g DTPE $(ADMA\ 8)_3$ zusetzt, erhält man ein analoges Resultat.

## Beispiel 11

Chypre-Komposition

| | Gewichtsteile |
|---|---|
| Vetiverylacetat | 200 |
| Bergamotteöl Kalabrien | 150 |
| Patchouliöl | 120 |
| Hydroxycitronellal | 100 |
| Linalool synthetisch | 100 |
| Sandelholzessenz | 60 |
| Phenyläthylalkohol | 60 |
| α-Methylionon | 50 |
| Ylang-Ylang-öl | 30 |
| Ketonmoschus | 30 |
| Amylsalicylat | 20 |
| "Rhodinol" (Citronellol ex Geraniumöl) | 20 |
| Eugenol extra | 10 |
| Limetteöl destilliert | 10 |
| Baummoos absolue 50% in Dipropylenglykol | 40 |
| | 1000 |

10 g dieser Komposition werden mit Aethanol auf 100 ml verdünnt. Das Gemisch ist gelb-orange gefärbt. 100 ml dieses Gemisches werden mit 2,5 g AeDTE $(ADMA\ 8)_2$ versetzt, wobei der Farbton von orange-gelb in hell-gelb umschlägt. Belichten dieses Gemisches erbringt weder eine geruchliche noch eine farbliche Veränderung.

## Beispiel 12

In 23,85 g der Chypre-Komposition des Beispiels 12 werden 0,15 g (0,5%) 4-(1,1-Dimethyläthyl)-4'-methoxydibenzoylmethan gelöst. Die ursprünglich gelb-braune Lösung färbt sich nach 2 bis 3 Minuten braun-rot. Darauf werden 6 g einer 5%igen Lösung von AeDTE $(ADMA\ 8)_2$ in Dipropylenglykol zugegeben (1% AeDTE $(ADMA\ 8)_2$), wobei die Komposition sofort eine gelbe Farbe annimmt.

## Beispiel 13

### Stabilisatorgemisch und dessen Wirksamkeit

| | Gewichtsteile |
|---|---|
| α-Tocopherol 50% | 20 |
| Ascorbylpalmitat | 50 |
| 4-(1,1-Dimethyläthyl)-4'-methoxydibenzoylmethan | 15 |
| AeDTE $(ADMA\ 8)_2$ | 15 |

Die Herstellung geschieht durch Zusammenmischen der Komponenten und Homogenisieren.

Das Präparat ist gut licht- und wärmestabil.

Es wird nun eine Chypre-base folgender Zusammensetzung erstellt:

| | |
|---|---|
| Vetiverylacetat | 200 |
| Bergamotteöl Kalabrien | 150 |
| Patchouliöl | 120 |
| Hydroxycitronellal | 100 |
| Linalool synthetisch | 100 |
| Sandelholzessenz | 60 |
| Phenyläthylalkohol | 60 |
| α-Irison (α-Methylionon) | 40 |
| Ylang-Ylang-öl | 30 |
| Ketonmoschus | 30 |
| Amylsalicylat | 20 |
| "Rhodinol" (Citronellol ex Geraniumöl) | 20 |
| Eugenol extra | 10 |
| Dione A (2-[3,3,5-Trimethyl-cyclohexylacetyl]-cyclopentanon 10% DPG | 10 |
| Limetteöl destilliert | 10 |
| Baummoos absolue 50% in DPG | 40 |
| | 1000 |

Zu der Chypre-base werden 3% des obigen Stabilisatorgemisches gegeben, und die entstandene Suspension 6 Stunden bei 20°C gerührt, hierauf durch Filtrieren geklärt.

farblicher Befund

| | ohne Stabilisator | mit 3% Stabilisator |
|---|---|---|
| nach Filtration | dunkelbraun | orangebraun |
| nach 4 Stunden belichten mit Xenonlampe 1100W/43°C | dunkelbraun | orangebraun |

die Signifikation dieser Aufhellung wird verdeutlicht durch:

Befund

| B | ohne Stabilisator | mit Stabilisator |
|---|---|---|
| nach Erstellen einer 10%-igen Lösung obiger Chyprebase | braun | hellgelb |
| nach 4 Stunden Belichten mit Xenonlampe 1100W/43°C | dunkelbraun | hellgelb |

Handelsübliche Colognes enthalten nun sogar nur ca. 5 - 6% einer Chyprekomposition der obigen Zusammensetzung, und, je verdünnter die Komposition, je eklatanter die Aufhellung.

## Patentansprüche

1. In alkoholischer Lösung praktisch neutral reagierende Salze sekundärer di-$C_{6-22}$-Alkylamine, und insbesondere tertiärer $C_{16-22}$-Alkyl-dimethyl- und di-$C_{6-22}$-Alkyl-$C_{1-2}$-alkylamine mit Aminopolycarbonsäuren.

2. Salze gemäss Anspruch 1, dadurch gekennzeichnet, dass das Amin ein tertiäres Fettamin, insbesondere ein Alkyl-dimethylamin ist.

3. Salze gemäss Anspruch 1 oder 2, dadurch gekennzeichnet, dass das tertiäre Fettamin Octadecyl-dimethylamin (ADMA 8) ist.

4. Salze gemäss Anspruch 1 oder 2, dadurch gekennzeichnet, dass das tertiäre Fettamin Oleyl-dimethylamin, Behenyldimethylamin oder Erucyldimethylamin ist.

5. Salze gemäss Anspruch 1 oder 2, dadurch gekennzeichnet, dass das Amin ein tertiäres di-$C_{6-22}$-Alkyl--$C_{1-2}$-alkylamin ist.

6. Salze gemäss Anspruch 1 oder 2, dadurch gekennzeichnet, dass das Amin ein sekundäres di-$C_{6-22}$-Alkylamin ist.

7. Salze gemäss Anspruch 6, dadurch gekennzeichnet, dass das sekundäre Fettamin Distearylamin (DSA) ist.

8. Salze gemäss Anspruch 6, dadurch gekennzeichnet, dass das sekundäre Fettamin Dihexylamin, Dioctylamin, Diisooctylamin (Di-(-2-äthylhexylamin) oder Dicyclohexylamin ist.

9. Salze gemäss einem der Ansprüche 1 bis 8, dadurch

gekennzeichnet, dass als Aminopolycarbonsäure Aethylendiamintetraessigsäure (AeDTE) verwendet wird.

10. Salze gemäss einem der Ansprüche 1 bis 8, dadurch gekennzeichnet, dass als Aminopolycarbonsäure Diäthylentriaminpentaessigsäure (DTPE) verwendet wird.

11. Salze gemäss einem der Ansprüche 1 bis 8, dadurch gekennzeichnet, dass als Aminopolycarbonsäure N-(2-Hydroxyäthyl)äthylendiamintriessigsäure (HAeDTE) verwendet wird.

12. AeDTE (ADMA 8)$_2$.

13. AeDTE (ADMA 8)$_3$

14. AeDTE (DSA)$_3$.

15. DTPE (ADMA 8)$_2$, DTPE (ADMA 8)$_3$ oder HAeDTE (ADMA 8)$_2$

16.Salze gemäss einem der Ansprüche 1 bis 15, zur Stabilisierung von Riechstoffkompositionen.

17. Riechstoffkompositionen, gekennzeichnet durch einen Gehalt an einem Salz gemäss einem der Ansprüche 1 bis 15.

18. Riechstoffkompositionen, gekennzeichnet durch einen Gehalt an einem Salz gemäss einem der Ansprüche 1 bis 15 und einem oder mehreren weiteren Hilfsstoffen.

19. Verfahren zur Herstellung der Salze gemäss einem der Ansprüche 1 bis 15, dadurch gekennzeichnet, dass man eine Aminopolycarbonsäure mit einem sekundären di-$C_{6-22}$-Alkylamin, oder insbesondere einem tertiären $C_{16-22}$-Alkyldimethyl- oder di-$C_{6-22}$-Alkyl-$C_{1-2}$-alkylamin neutralisiert.

20. Verfahren zur Stabilisierung von Riechstoffkompositionen, dadurch gekennzeichnet, dass man zu diesem Zweck ein Salz gemäss einem der Ansprüche 1 bis 15 verwendet.

21. Verfahren gemäss Anspruch 20, dadurch gekennzeichnet, dass man zusätzlich einen oder mehrere weitere Hilfsstoffe verwendet.

22. Verwendung der Salze gemäss einem der Ansprüche 1 bis 15, zur Stabilisierung von Riechstoffkompositionen.

23. Verwendung der Salze gemäss einem der Ansprüche 1 bis 15, in Kombination mit einem oder mehreren weiteren Hilfsstoffen

Europäisches Patentamt

## EUROPÄISCHER RECHERCHENBERICHT

0170863

Nummer der Anmeldung

EP 85 10 7969

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | EP-A-0 077 406 (TAKEMOTOYUSHI)<br>* Ansprüche; Seiten 6-9 *<br>--- | 1,19 | C 07 C 101/02<br>C 07 C 87/127<br>C 07 C 87/24<br>A 61 K 7/46 |
| Y | FR-A-2 372 850 (AIR PRODUCTS)<br>* Ansprüche; Seite 3 *<br>--- | 1 | |
| Y | GB-A-1 077 458 (J.R. GREIGY)<br>* Ansprüche *<br>----- | 1,20-23 | |
| | | | RECHERCHIERTE SACHGEBIETE (Int. Cl.4) |
| | | | C 07 C 101/00<br>C 07 C 87/00<br>A 61 K 7/00 |

Der vorliegende Recherchenbericht wurde für alle Patentansprüche erstellt.

| Recherchenort | Abschlußdatum der Recherche | Prüfer |
|---|---|---|
| DEN HAAG | 21-10-1985 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument